# FASCICULE DE BREVET EUROPEEN

(11) **EP 0 975 316 B1**
(45) Date de publication et mention de la délivrance du brevet: **15.09.2004**
(21) Numéro de dépôt: 98963625.3
(22) Date de dépôt: 23.12.1998
(51) Int. Cl.: A61K 7/06, A61K 7/48

(54) **COMPOSITION COSMETIQUE A BASE DE POLYURETHANNES ASSOCIATIFS ET DE POLYMERES NON IONIQUES A CHAINES GRASSES**
KOSMETISCHE ZUSAMMENSETZUNG AUF DER BASIS VON ASSOZIATIVEN POLYURETHANEN UND NICHTIONISCHEN POLYMEREN MIT FETTKETTEN
COSMETIC COMPOSITION BASED ON ASSOCIATIVE POLYURETHANE AND NON-IONIC POLYMERS WITH FATTY CHAINS

(30) Priorité: 13.02.1998 FR 9801777
(43) Date de publication de la demande: 02.02.2000
(60) Demande divisionnaire: 04290610.7
(73) Titulaire: L'OREAL, 75008 Paris (FR)
(72) Inventeur: DUPUIS, Christine, F-75018 Paris (FR)
(74) Mandataire: Miszputen, Laurent
(86) Numéro de dépôt international: PCT/FR1998/002866
(87) Numéro de publication internationale: WO 1999/040893

(56) Documents cités:
- EP-A- 0 339 712
- EP-A- 0 637 600
- EP-A- 0 648 485
- EP-A- 0 745 373
- EP-B- 0 555 155
- WO-A-97/48377
- WO-A-98/00499
- WO-A-99/36047
- DE-A- 4 438 846
- FR-A- 2 745 173
- US-A- 5 508 454

## Description

La présente invention concerne des compositions cosmétiques contenant un nouveau système épaississant de milieux aqueux à base de polyuréthannes associatifs et de polymères non ioniques à chaînes grasses, ainsi que leur utilisation en tant que gels coiffants capillaires non rincés.

L'épaississement et/ou la gélification des milieux aqueux par des polymères est depuis longtemps un important de sujet de recherche cosmétique. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

Une approche intéressante a consisté à utiliser comme épaississants des polymères capables de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis.

De tels polymères capables de s'associer réversiblement entre eux ou avec d'autres molécules sont appelés "polymères associatifs". Les forces d'interactions en jeu peuvent être de nature très différente par exemple de nature électrostatique, de type liaisons hydrogène ou des interactions hydrophobes.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes par lesquelles les polymères interagissent et se rassemblent entre eux ou avec d'autres molécules.

Il est connu de préparer des compositions capillaires sous forme de gel utilisant, comme système épaississant, de tels polymères amphiphiles associatifs, en conjonction avec des agents tensioactifs. On pense que les propriétés rhéologiques intéressantes des gels ainsi obtenus sont dues à la formation de micelles mixtes contenant les agents tensioactifs et les parties hydrophobes des polymères amphiphiles, ces micelles constituant une multitude de points de réticulation physique.

Cependant, ces compositions à base de polymères associatifs et d'agents tensioactifs n'ont pas toujours les propriétés cosmétiques souhaitées. Ainsi, la présence d'agents tensioactifs, même en faibles quantités, peut modifier de façon indésirable les propriétés cosmétiques desdites compositions, telles que les propriétés d'application ou de toucher après séchage. Par ailleurs, notamment dans le domaine des gels de coiffage ou de soin non rincés, il est important de pouvoir répartir uniformément le produit sur l'ensemble de la chevelure de manière à éviter les surcharges et les défauts cosmétiques qui en résultent.

Le document EP 555 155 vise l'obtention d'une mousse douce, conférant un bon démêlage, au moyen d'une composition contenant un agent tensioactif non ionique de type alkylpolyglycoside et/ou un agent tensioactif non ionique polyglycérolé et un uréthanne-polyéther.

Le document W098/00499 vise l'obtention d'un produit de nettoyage, formant une mousse au moyen d'un système épaississant formé par un polymère associatif et une huile polaire de HLB supérieur à 12, des tensioactifs et de l'eau.

Les documents précités ne dévoilent pas la formulation de la présente invention.

On a découvert à présent qu'il était possible d'obtenir un bon effet épaississant, voire gélifiant, et des propriétés cosmétiques intéressantes en associant des polyuréthannes associatifs non ioniques à des polymères non ioniques comportant au moins un motif à chaîne grasse.

Le gel obtenu par l'association de ces deux types de polymères a une texture très fondante et est agréable à appliquer. Le toucher final sur cheveux séchés est plus agréable et moins chargé. Le gel a par ailleurs un excellent pouvoir coiffant.

Un objet de la présente invention est donc une composition cosmétique comprenant au moins un polyuréthanne non ionique associatif en combinaison avec au moins un polymère non ionique comportant au moins un motif à chaîne grasse.

Un autre objet de la présente invention est l'utilisation de la combinaison d'au moins un polyuréthanne non ionique associatif et d'au moins un polymère non ionique comportant au moins un motif à chaîne grasse en tant que système épaississant pour des compositions cosmétiques.

Un troisième objet de l'invention est un procédé de traitement cosmétique des cheveux utilisant une composition cosmétique obtenue par association d'au moins un polyuréthanne associatif non ionique et d'au moins un polymère non ionique comportant au moins un motif à chaîne grasse.

D'autres objets apparaîtront à la lecture de la description et des exemples qui suivront.

Les compositions cosmétiques conformes à l'invention sont essentiellement caractérisées par le fait qu'elles contiennent, dans un milieu cosmétiquement acceptable,
(A) au moins un polyuréthanne associatif non ionique correspondant à la formule générale 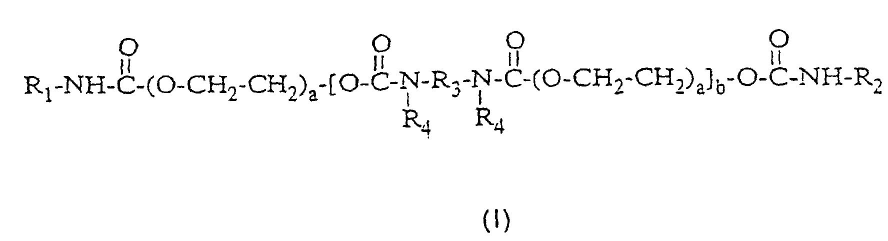 dans laquelle
   au moins un des résidus R₁ et R₂ représente un groupe alkyle supérieur en C₈₋₁₈ et l'autre, le cas échéant, un groupe alkyle inférieur en C₁₋₆,
   R₃ représente un radical hydrocarboné en C₄₋₃₆, de préférence en C₆₋₁₀,
   R₄ représente un atome d'hydrogène ou un radical alkyle en C₁₋₆, de préférence un atome d'hydrogène,
   a varie indépendamment de 90 à 600, et
   b vaut de 1 à 4, et
(B) au moins un polymère non ionique comportant au moins un motif à chaîne grasse et qui est différent du polyuréthanne de formule (I).

On entend par groupe alkyle inférieur en C₁₋₆, selon l'invention, un groupe alkyle à chaîne linéaire ou ramifiée comportant de 1 à 6 atomes de carbone, tel que les radicaux méthyle, éthyle, n-propyle, n-butyle,
*n*-pentyle et *n*-hexyle ainsi que les isomères ramifiés correspondants.

Conformément à l'invention, les groupes alkyle supérieurs en
C₈₋₁₈ désignent des groupes alkyle à chaîne linéaire ou ramifiée comportant de 8 à 18 atomes de carbone, tels que les radicaux octyle, nonyle, décyle, undécyle, dodécyle, tridécyle, tétradécyle, pentadécyle, hexadécyle, heptadécyle et octadécyle.

Dans un mode de réalisation préféré, un seul des radicaux alkyle R₁ et R₂ en a-W du polymère de formule (I) représente un groupe alkyle supérieur en C₈₋₁₈ et l'autre un groupe alkyle inférieur en C₁₋₆.

Un polyuréthanne associatif particulièrement recommandé de la présente invention est celui dans lequel un des groupes R₁ et R₂ représente un radical octadécyle et l'autre un groupe méthyle.

Les polyuréthannes associatifs utilisés dans les compositions de la présente invention sont utilisés sous forme de solution ou suspension aqueuse contenant éventuellement une certaine quantité d'amidon soluble. Cet amidon peut être n'importe quel amidon extrait de sources naturelles, tel que l'amidon de blé, de maïs, de riz, de pomme de terre etc., et qui a été modifié par voie chimique, enzymatique ou microbiologique de manière à être soluble dans l'eau.

Un polymère préféré est commercialisé par la Société Rohm & Haas sous la dénomination ACRYSOL 46. Il s'agit d'un polyuréthanne obtenu par condensation d'hexaméthylènediisocyanate et de polyéthylèneglycol, et portant à ses extrémités respectivement en moyenne un résidu méthyle et un résidu octadécyle. Ce polymère se présente sous forme d'une solution aqueuse à 15 % en poids de matière active polyuréthanne contenant, en plus, de 3 - 5 % d'une matrice d'amidon modifié par voie enzymatique.

Les polymères non ioniques comportant au moins une chaîne grasse selon la présente invention, utilisés comme composant (B), sont choisis parmi :
les copolymères de méthacrylates ou d'acrylates hydrophiles et de monomères hydrophobes comportant au moins une chaîne grasse en C₈₋₂₂ tels que par exemple un copolymère de méthacrylate de polyéthylèneglycol et de méthacrylate de lauryle comme le produit 470/66 commercialisé par la société NATIONAL STARCH.

Selon l'invention, les polyuréthannes associatifs et les polymères non ioniques à chaînes grasses sont utilisés en des quantités suffisantes pour obtenir un épaississement ou une gélification satisfaisante du milieu aqueux.

On recommande notamment une quantité de polyuréthannes associatifs comprise entre 0,1 et 10 % en poids et, de préférence, entre 0,5 et 5 % en poids exprimé en matière active et rapportée au poids total de la composition.

Dans les compositions de la présente invention, les polymères non ioniques comportant au moins un motif à chaîne grasse sont présents à raison de 0,01 à 10 % en poids, de préférence à raison de 0,1 à 5 % en poids de matière active par rapport au poids total de la composition.

Dans la présente invention , le rapport dudit polyuréthanne associatif non ionique (A) de formule (I) audit polymère non ionique comportant au moins un motif à chaîne grasse (B) est compris de préférence dans l'intervalle allant de 90/10 à 10/90.

Le milieu cosmétiquement acceptable est constitué de préférence d'eau et peut contenir en outre des solvants cosmétiquement acceptables, par exemple des monoalcools inférieurs tels que l'éthanol ou l'isopropanol, des glycols tels que le diéthylèneglycol, des éthers de glycols tels que les alkyléthers d'éthylèneglycol ou de diéthylèneglycol, ou encore des esters d'acides gras, tous ces solvants étant utilisés seuls ou sous forme de mélange.

Les gels coiffants peuvent contenir en outre un ou plusieurs additifs utilisés habituellement dans de telles compositions capillaires. On peut citer à titre d'exemple les parfums, colorants, conservateurs, filtres solaires, vitamines, agents régulateurs de pH etc. Il est bien entendu que le choix de ces composés doit tenir compte d'éventuelles interactions avec le système épaississant. L'homme du métier veillera à ce que l'adjonction de ces additifs n'ait pas d'influence défavorable sur les propriétés avantageuses des compositions obtenues grâce à la présente invention.
Un procédé de traitement cosmétique des cheveux préféré, selon l'invention, consiste à appliquer et à répartir de façon homogène les compositions décrites ci-dessus sur les cheveux et à sécher les cheveux ainsi traités sans les rincer.

Les exemples suivants sont destinés à illustrer l'invention sans pour autant présenter un caractère limitatif.

### Exemple 1

On a préparé un gel ayant la composition suivante :

| | |
|---|---|
| ACRYSOL 46 | 2 % de matière active |
| 470/66* | 2 % de matière active |
| eau déminéralisée | q. s. p. 100 g |

| | |
|---|---|
| * Le polymère 470/66 (commercialisé par la société NATIONAL STARCH) est un copolymère de méthacrylate de polyéthylèneglycol et de méthacrylate de lauryle en solution aqueuse à 39,2 %. | |

Il est bien entendu que la description qui précède n'a été donnée qu'à titre purement illustratif et non limitatif et que des variantes ou des modifications peuvent y être apportées dans le cadre de la présente invention.

## Revendications

1. Composition cosmétique sous forme de Gel coiffant non rincé **caractérisée par le fait qu'**elle comprend, dans un milieu cosmétiquement acceptable,
(A) au moins un polyuréthanne associatif non ionique correspondant à la formule générale 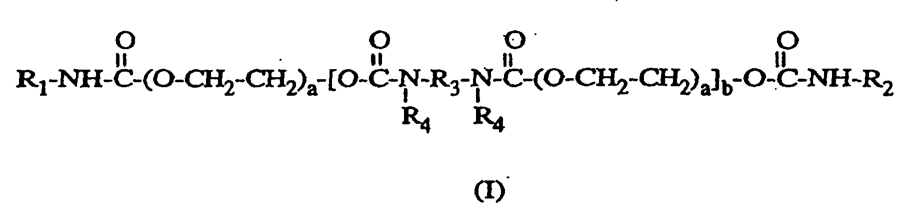 dans laquelle
au moins un des radicaux R₁ et R₂ représente un groupe alkyle supérieur en C₈₋₁₈ et l'autre, le cas échéant, un groupe alkyle inférieur en C₁₋₆, R₃ représente un radical hydrocarboné en C₄₋₃₆, de préférence en C₆₋₁₀, R₄ représente un atome d'hydrogène ou un radical alkyle en C₁₋₆, de préférence un atome d'hydrogène,
a varie indépendamment de 90 à 600, et
b vaut de 1 à 4, et
(B) au moins un polymère non ionique comportant au moins un motif à chaîne grasse et qui est différent du polymère de formule (1), choisi parmi les copolymères de méthacrylates ou d'acrylates hydrophiles et de monomères hydrophobes comportant au moins une chaîne grasse en C₈₋₂₂.

2. Composition cosmétique selon la revendication 1, **caractérisée en ce que** le composant (A) est un polyuréthanne associatif non ionique dans lequel un seul des radicaux R₁ et R₂ en a-w représente un groupe alkyle supérieur et l'autre un groupe alkyle inférieur.

3. Composition cosmétique selon la revendication 2, **caractérisée en ce que** le composant (A) est un polyuréthanne associatif non ionique dans lequel en moyenne un des radicaux R₁ et R₂ représente un groupe octadécyle et l'autre un groupe méthyle.

4. Composition cosmétique selon l'une quelconque des revendications 1 à 3, **caractérisée par le fait que** le composant (A) se présente sous forme d'une solution ou suspension dans l'eau contenant également de l'amidon soluble modifié par voie chimique, enzymatique ou microbiologique.

5. Composition selon l'une quelconque des revendications 1 à 4, **caractérisée en ce qu'**elle contient le composant (A) à raison de 0,1 à 10 % en poids, et de préférence à raison de 0,5 à 5 % en poids, exprimé en matière active rapportée au poids total de la composition.

6. Composition selon l'une quelconque des revendications 1 à 5, **caractérisée en ce qu'**elle contient le composant (B) à raison de 0,01 à 10 % en poids, de préférence à raison de 0,1 à 5 % en poids de matière active par rapport au poids total de la composition.

7. Composition selon l'une quelconque des revendications 1 à 6 **caractérisée par le fait que** le rapport en poids dudit polyuréthanne associatif non ionique de formule (I) audit polymère non ionique comportant au moins un motif à chaîne grasse est compris dans l'intervalle allant de 90/10 à 10/90.

8. Utilisation de l'association d'un polyuréthanne associatif non ionique de formule (I) et d'un polymère non ionique comportant au moins un motif monomère à chaîne grasse, choisi parmi, les copolymères de méthacrylates ou d'acrylates hydrophiles et de monomères hydrophobes comportant au moins une chaîne grasse en C₈₋₂₂, en tant que système épaississant d'une composition cosmétique.

9. Procédé de traitement cosmétique des cheveux, **caractérisé par le fait que** l'on applique sur les cheveux une composition définie selon l'une quelconque des revendications 1 à 8 et en ce que l'on sèche les cheveux ainsi traités sans les rincer.

## Patentansprüche

1. Kosmetische Zusammensetzung, die als Frisiergel vorliegt, das nicht ausgespült wird, **dadurch gekennzeichnet, dass** sie in einem kosmetisch akzeptablen Medium enthält:
(A) mindestens ein nichtionisches assoziatives Polyurethan der folgenden allgemeinen Formel: 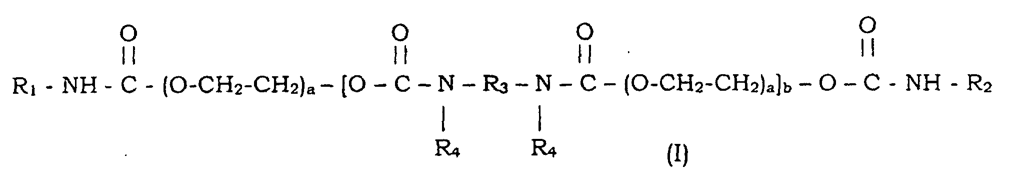 worin bedeuten:
mindestens eine der Gruppen R₁ und R₂ eine höhere C₈₋₁₈-Alkylgruppe , wobei die andere gegebenenfalls eine niedere C₁₋₆-Alkylgruppe bedeutet;
R₃ eine C₄₋₃₆-Kohlenwasserstoffgruppe und vorzugsweise eine C₆₋₁₀-Kohlenwasserstoffgruppe,
R₄ ein Wasserstoffatom oder eine C₁₋₆-Alkylgruppe und vorzugsweise ein Wasserstoffatom,
a liegt im Bereich von 90 bis 600 und
b im Bereich von 1 bis 4, und
(B) mindestens ein nichtionisches Polymer, das mindestens eine Fettkette aufweist und von dem Polymer der Formel (I) verschieden ist und das unter den Copolymeren von hydrophilen Methacrylaten oder Acrylaten und hydrophoben Monomeren, die mindestens eine C₈₋₂₂-Fettkette enthalten, ausgewählt ist.

2. Kosmetische Zusammensetzung nach Anspruch 1, **dadurch gekennzeichnet, dass** die Komponente (A) ein nichtionisches assoziatives Polyurethan ist, in dem nur eine der Gruppen R₁ und R₂ in a-w-Stellung eine höhere Alkylgruppe bedeutet und die andere Gruppe eine niedere Alkylgruppe ist.

3. Kosmetische Zusammensetzung nach Anspruch 2, **dadurch gekennzeichnet, dass** die Komponente (A) ein nichtionisches assoziatives Polyurethan ist, worin im Mittel eine der Gruppen R₁ und R₂ eine Octadecylgruppe bedeutet und die andere Gruppe eine Methylgruppe ist.

4. Kosmetische Zusammensetzung nach einem der Ansprüche 1 bis 3, **dadurch gekennzeichnet, dass** die Komponente (A) als Lösung oder Suspension in Wasser vorliegt, die auch eine auf chemischem, enzymatischen oder mikrobiologischen Wege modifizierte lösliche Stärke enthält.

5. Zusammensetzung nach einem der Ansprüche 1 bis 4, **dadurch gekennzeichnet, dass** sie die Komponente (A) in einer Menge von 0,1 bis 10 Gew.-% und vorzugsweise 0,5 bis 5 Gew.-% enthält, wobei der Mengenanteil als Wirkstoff bezogen auf das Gesamtgewicht der Zusammensetzung ausgedrückt ist.

6. Zusammensetzung nach einem der Ansprüche 1 bis 5, **dadurch gekennzeichnet, dass** sie die Komponente (B) in einer Wirkstoffmenge von 0,01 bis 10 Gew.-% und vorzugsweise 0,1 bis 5 Gew.-%, bezogen auf das Gesamtgewicht der Zusammensetzung, enthält.

7. Zusammensetzung nach einem der Ansprüche 1 bis 6, **dadurch gekennzeichnet, dass** das Gewichtsverhältnis des nichtionischen assoziativen Polyurethan der Formel (I) und des nichtionischen Polymers, das mindestens eine Einheit mit Fettkette aufweist, im Bereich von 90/10 bis 10/90 liegt.

8. Verwendung eines nichtionischen assoziativen Polyurethan der Formel (I) in Kombination mit einem nichtionischen Polymer, das mindestens eine Monomereinheit mit Fettkette enthält und unter den Copolymeren von hydrophilen Acrylaten oder Methacrylaten und hydrophoben Monomeren mit mindestens einer C₈₋₂₂-Fettkette ausgewählt ist, als Verdickungssystem einer kosmetischen Zusammensetzung.

9. Verfahren zur kosmetischen Behandlung der Haare, **dadurch gekennzeichnet, dass** auf die Haar eine Zusammensetzung nach einem der Ansprüche 1 bis 8 aufgebracht wird und die so behandelten Haare getrocknet werden, ohne dass sie ausgespült werden.

## Claims

1. Cosmetic composition in the form of a leave-in styling gel, **characterized in that** it comprises in a cosmetically acceptable medium,
(A) at least one nonionic associative polyurethane corresponding to the general formula 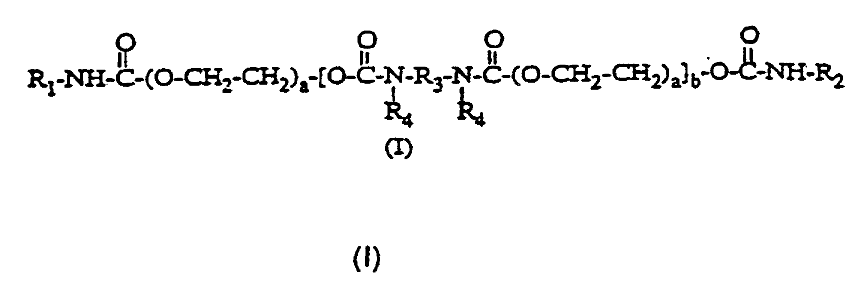 in which
at least one of the radicals R₁ and R₂ represents a C₈-C₁₈ higher alkyl group and the other, where appropriate, represents a C₁-C₆ lower alkyl group,
R₃ represents a C₄-C₃₆, preferably C₆-C₁₀, hydrocarbon-based radical,
R₄ represents a hydrogen atom or a C₁-C₆ alkyl radical, preferably a hydrogen atom,
a ranges, independently, from 90 to 600, and
b is from 1 to 4, and
(B) at least one nonionic polymer comprising at least one unit containing a fatty chain and which is other than the polymer of formula (I) chosen from copolymers of hydrophilic methacrylates or acrylates and of hydrophobic monomers comprising at least one C₈₋₂₂ fatty chain.

2. Cosmetic composition according to Claim 1, **characterized in that** the component (A) is a nonionic associative polyurethane in which only one of the a-w radicals R₁ and R₂ represents a higher alkyl group and the other represents a lower alkyl group.

3. Cosmetic composition according to Claim 2, **characterized in that** the component (A) is a nonionic asociative polyurethane in which on average one of the radicals R₁ and R₂ represents an octadecyl group and the other represents a methyl group.

4. Cosmetic composition according to any one of Claims 1 to 3, **characterized in that** the component (A) is in the form of a solution or suspension in water also containing chemically, enzymatically or microbiologically modified soluble starch.

5. Composition according to any one of Claims 1 to 4, **characterized in that** it contains the component (A) in a proportion of from 0.1 to 10 % by weight, and preferably in a proportion of from 0.5 to 5 % by weight, expressed in active material relative to the total weight of the composition.

6. Composition according to any one of Claims 1 to 5, **characterized in that** it contains the component (B) in a proportion of from 0.01 to 10 % by weight, preferably in a proportion of from 0.1 to 5 % by weight, of active material relative to the total weight of the composition.

7. Composition according to any one of Claims 1 to 6, **characterized in that** the weight ratio between the said nonionic associative polyurethane of formula (I) and the said nonionic polymer comprising at least one unit containing a fatty chain is within the range from 90/10 to 10/90.

8. Use of the combination of a nonionic associative polyurethane of formula (I) and of a nonionic polymer comprising at least one monomer unit containing a fatty chain chosen from copolymers of hydrophilic methacrylates or acrylates and of hydrophobic monomers comprising at least one C₈₋₂₂ fatty chain, as a system for thickening a cosmetic composition.

9. Cosmetic process for treating the hair, **characterized in that** a composition defined according to any one of Claims 1 to 8 is applied to the hair and **in that** the hair thus treated is dried without rinsing it.
